# EUROPEAN PATENT APPLICATION

(11) **EP 1 111 043 A1**
(43) Date of publication of application: **27.06.2001**
(21) Application number: 99204461.0
(22) Date of filing: 21.12.1999
(51) Int. Cl.: C12N 15/11, C12N 5/10, A01K 67/02, A01K 67/027, C12Q 1/68

(54) **New QTL's on chromosomes X, 2, 6 and 7 of pigs**

(71) Applicant: Institute for Pig Genetics B.V., 6641 SZ Beuningen (NL); NUTRECO NEDERLAND B.V., 5830 AE Boxmeer (NL)
(72) Inventor: DE KONING, Dirk-Jan, 6871 BM Renkum (NL); GROENEN, Martinus Antonius Mathilda, 6671 BK Zetten (NL); VAN ARENDONK, Johannus Antonius Maria, 6721 GP Bennekom (NL)
(74) Representative: Ottevangers, Sietse Ulbe

(57) **Abstract**

The invention relates to the field of breeding domestic animals, in particular pigs.

The invention provides an isolated and/or recombinant nucleic acid comprising a sequence affecting the quantitative trait muscle depth in mammals, said sequence being derived from a locus corresponding to region HSA6p21.3-p.22 in humans and corresponding to the homologous region on ssc7 in pigs, in particular wherein said quantitative trait is maternally expressed.

Also, the invention provides an isolated and/or recombinant nucleic acid comprising a sequence affecting the quantitative trait back fat thickness in mammals, said sequence being derived from a locus corresponding to a region on ssc7 in pigs, which region overlaps with the region identified in claim 1 affecting the quantative trait muscle depth on ssc 7 in pigs, and an isolated and/or recombinant nucleic acid comprising a sequence affecting the quantitative trait back fat thickness in mammals, said sequence being derived from a locus corresponding to a region on ssc2 of pigs which maps about 35 cM away from the IGF2 region, in particular wherein said quantitative trait is paternally expressed.

## Description

The present invention relates to the field of breeding domestic animals, in particular pigs. In particular it relates to genotypic and/or phenotypic traits which need to be selected for in breeding animals or animals intended for slaughter. Traits having to do with desired phenotypic properties in domestic animals, such as backfat, muscle depth, growth rate and feed conversion are often referred to as quantitative traits. In recent years efforts have been directed at identifying the genomic localisation of such quantitative traits, e.g. by means of microsatellite markers. The resulting localisation leads to identification of so-called quantitative trait loci (QTL). Identifying quantitative trait loci is an important part of arriving at means for selecting suitable breeding animals having desired genotypic and/or phenotypic properties, but also very important is determining the way of inheritance of such traits into offspring. The present invention provides a number of new quantitative trait loci and also provides information on their inheritance pattern, thereby providing new methods and means for selecting animals for breeding purposes and/or slaughtering and for implementing marker assisted breeding methods and the like.

Thus the present invention provides an isolated and/or recombinant nucleic acid comprising a sequence affecting the quantitative trait muscle depth in mammals, said sequence being derived from a locus corresponding to region HSA6p21.3-p.22 in humans and corresponding to the homologous region on ssc7 in pigs, wherein said quantitative trait locus is maternally expressed.

The invention further provides an isolated and/or recombinant nucleic acid comprising a sequence affecting the quantitative trait back fat thickness in mammals, said sequence being derived from a locus corresponding to a region on ssc7 in pigs, which region overlaps with the region identified in claim 1 affecting the quantitative trait muscle depth on ssc7 in pigs. This QTL inherits in a Mendelian fashion.

In yet another embodiment the invention provides an isolated and/or recombinant nucleic acid comprising a sequence affecting the quantitative trait back fat thickness in mammals, said sequence being derived from a locus corresponding to a region on ssc2 of pigs which maps about 35 cM away from the IGF2 region, wherein said quantitative trait locus is paternally expressed.

In another embodiment the invention provides an isolated and/or recombinant nucleic acid comprising the sequence of a maternally expressed quantitative trait locus affecting intramuscular fat in mammals, said sequence being derived from a locus corresponding to a region on the long arm of ssc6 of pigs.

In yet another embodiment the invention provides an isolated and/or recombinant nucleic acid comprising a sequence of the paternally expressed quantitative trait locus affecting intramuscular fat in mammals, said sequence being derived from a region HSA6q22-ter in humans and corresponding to the homologous on the short arm of ssc6 of pigs. This trait is inherited in a Mendelian fashion.

The recombinant nucleic acids according to the invention can be used in breeding programs in the same manner, QTL's have been used in selection methods so far. Care should be taken however of the fashion in which the traits inherit. Combining Mendelian traits and imprinted traits will give rise to different selections and breeding programmes than relying on either one of these. It provides tools to arrive at improved properties in an easier, more reproducible way. The QTL's according to the invention affect a number of similar traits and can thus be ideally combined for just such a purpose. The nucleic acids according to the invention can of course also be applied in the production of transgenic animals if such is desired.

Furthermore based on the identified QTL's isolated nucleic acids can be (synthetically or recombinantly) prepared to be used to detect these traits in animals or samples from animals, in particular mammals, especially pigs. For detecting such traits using any hybridisation technique, including amplification, in situ hybridisation and the like, such isolated nucleic acids can be used. They may be labelled for such a purpose, during, before or after the hybridisation step, in order to allow for easy detection. Thus the invention also provides an isolated and/or recombinant nucleic acid according to the invention which is a specific probe or primer for a quantitative trait and/or an isolated and/or recombinant nucleic acid comprising a sequence capable of identifying a quantitative trait as identified herein before. Typically such a nucleic acid may be based on, or derived from a microsatellite marker, that is located near the actual QTL. The invention of course also provides the use of an isolated and/or recombinant nucleic acid according to the invention in identifying a quantitative trait locus in a mammal, in particular also when said quantitative trait locus is parentally imprinted.

The invention also provides the use of an isolated and/or recombinant nucleic acid acoording to the invention for selection of a domestic animal having desired genetic properties. Taking a sample from an animal and testing it with a nucleic acid according to the invention is well within the skill of the art and needs no further explanation here. Selection of the desired allele is also within the art, e.g. using mismatch PCR and other techniques for distinguishing alleles.

The invention also includes these uses wherein said domestic animal is a breeding animal or an animal for slaughter. Of course the results of testing in particular breeding animals for desired properties will be put to, good use in breeding programmes. Thus the invention also includes methods for breeding a population of domestic animals having desired genetic properties, comprising testing animals to be cross-bred with at least one nucleic acid according to the invention, for the presence of a desired allele of a quantitative trait, in particular where such a method comprises a method wherein said testing comprises contacting a sample from an animal to be cross-bred with a nucleic acid according the invention and detecting the presence or absence of hybridisation. Cells and animals provided with a nucleic acid according to the invention are also provided by the present invention. Thus the invention provides a mammalian cell comprising a recombinant nucleic acid corresponding to a QTL as defined according to the invention, as well as a transgenic animal comprising at least one cell as disclosed above.

### Detailed description

Recently imprinting at the *IGF2* locus in two different pig crosses^{1,2},was disclosed. We have investigated the role of imprinting in an intercross between Chinese Meishan and commercial Dutch pigs. On 785 F₂ animals we recorded three body composition traits after slaughter: backfat thickness (BFT), muscle depth (MD), and the percentage of intramuscular fat (IMF) inside the *Musculus longissimus*^{*3*}. An earlier analysis⁴ of BFT and IMF showed significant evidence for QTLs on chromosomes 2 and 7, ignoring effects of imprinting.

A whole genome scan using 132 microsatellite markers was used to map autosomal QTLs on the F₂ population, including a test for imprinting. The statistical model for imprinting⁵ was reparameterized to separate the contribution of the paternally and maternally inherited effect. Mode of inheritance for a putative QTL was inferred from the variance explained by the components in the model. Our genome scan resulted in five significant QTLs affecting body composition traits, of which four were imprinted.

There was strong evidence for a paternally expressed QTL for BFT on chromosome 2 (Table 1). For the QTL affecting BFT on chromosome 7, both the paternal and maternal component were highly significant implying Mendelian expression for this QTL. Also on chromosome 7, a highly significant QTL for MD mapped to the same area as the QTL for BFT. In contrast to the QTL for BFT, however, the QTL for MD was maternally expressed (Table 1). From these results, it cannot be distinguished whether there are two separate loci or one pleiotropic locus that shows imprinting during one stage of development and Mendelian expression during another.

With a model ignoring imprinting³, suggestive evidence for a Mendelian QTL for IMF was found on the long arm of chromosome 6. The present analysis, however, revealed that this was caused by a paternally expressed QTL (Table 1). In addition, a maternally expressed QTL was found on the short arm of the same chromosome.

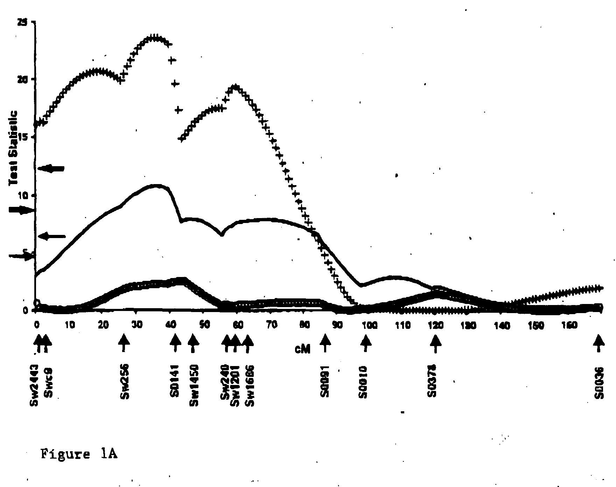

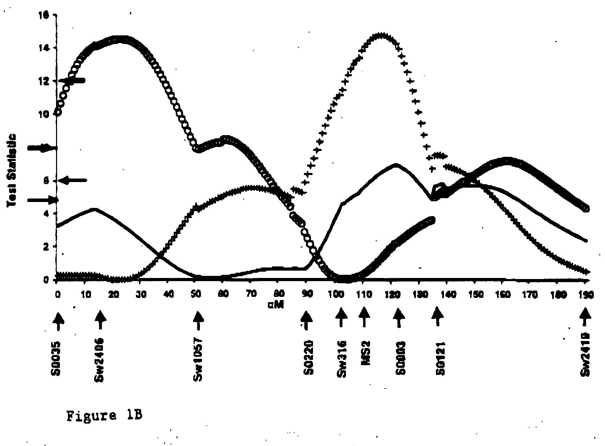

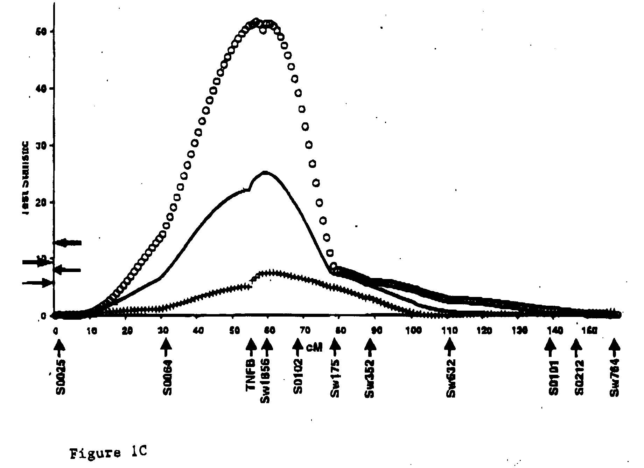

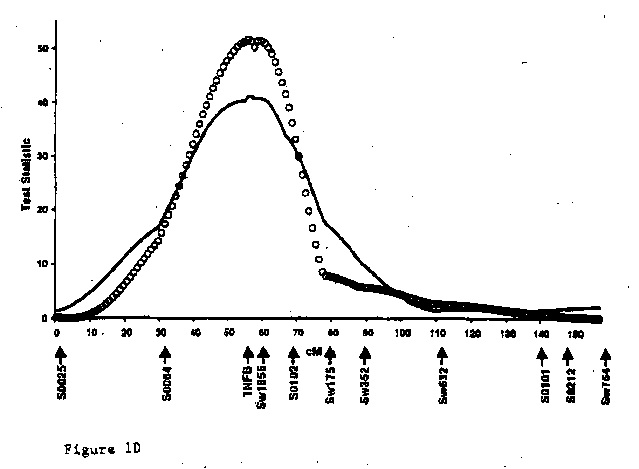

A graphical comparison of results obtained under the imprinting and Mendelian models is given in Fig 1. The imprinted QTL on chromosome 2 maps at about 35 cM away from the *IGF2* region, for which there is an imprinted QTL for body composition¹

The general outline of the comparative map between pig and human for the regions of interest^{6,7} has been established and is shown in Fig. 1. Genes that have been mapped more precisely in the pig by linkage analysis or on the radiation hybrid panel⁹ facilitated refinement of the comparative map. We realize that the comparative map presented here is not comprehensive, and that some genes originating from other chromosomes are reported but not represented in Fig. 1.

The QTL for MD on SSC7 can be narrowed to a region homologous with HSA6p21.3-p22. This region contains the major histocompatibility complex including *LTA* and shows extensive conservation in gene order¹⁰. Imprinted genes have not been reported for this region in humans and mice¹¹
(http://www.mgc.har.mrc.ac.uk/imprinting/imptables.html).

Several genes that map to the area of the maternally expressed QTL on SSC6p are located on HSA 16q22-ter, where imprinting has not been reported either. The area within the confidence interval of the paternally expressed QTL for IMF on SSC6q, however, extends on both sides to homologous regions in humans, where imprinted genes have been reported (*PEG3* on HSA 19q13.4 is imprinted in mice and *p73* on HSA 1p36 is maternally expressed). Genes *MC5R*, *FABP3*^{*12*} and *UOX*^{*9*} within the interval SW316 - S0003 covering the peak of the QTL, are located on HSA18p11.2, 1p33-p32, and 1p22, respectively. Imprinting has not been found for these candidate regions in humans. For chromosome 2, imprinting is reported for the *IGF2* area but until now homology to other imprinting clusters cannot be established clearly. Data on imprinting of Wilms Tumor 1 (*WT1*) on HSAllpl3 demonstrate that imprinting can be specific for certain tissues and developmental stages¹¹

The X chromosome harbors quantitative trait loci for backfat thickness and intramuscular fat content in pigs

Genetic markers have been used in experimental crosses in pigs to dissect quantitative trait variation (e.g. Andersson et al. 1994, Knott et al. 1998, Rohrer and Keele, 1998, Walling et al. 1998). We reported recently on the mapping of autosomal quantitative trait loci (QTL) for fatness traits in an experimental intercross between the obese Chinese Meishan breed and Dutch White production lines^{4,15}. In this paper, the analysis of the X chromosome on the total population described by Janss et al. (1997) is presented.

Briefly, 19 Meishan boars were mated to 120 sows of five different Large White and Landrace lines from Dutch breeding companies. From the F1 animals, 39 F1 and 124 F1 sows were randomly selected to generate the F2 generation. The F2 animals (n=1293), their F1 parents (n=303) and the 19 Meishan grandfathers were genotyped for 5 microsatellite markers located in the non-pseudoautosomal region of the X chromosome. The amount of fat within the muscle (intramuscular fat IMF) and backfat thickness (BFT) of the *musculus longissimus* was recorded on 785 F2 animals after slaughter at a live weight of approximately 90 kg³. For QTL analysis, interval mapping by regression was carried out as a line cross analysis as described previously⁴ where the founder lines were assumed to be fixed for different QTL alleles. The model was modified to account for differences between sexes. According to the design of the cross, male F2 offspring carry only a maternal copy of the X chromosome originating from the Meishan or from the White grandparents, whereas all female offspring inherited in addition the paternal copy of the X chromosome originating from the White lines. Therefore, the contrast between the Meishan and the White allele for the estimation of the QTL effect was estimated within male and female F2 offspring separately.

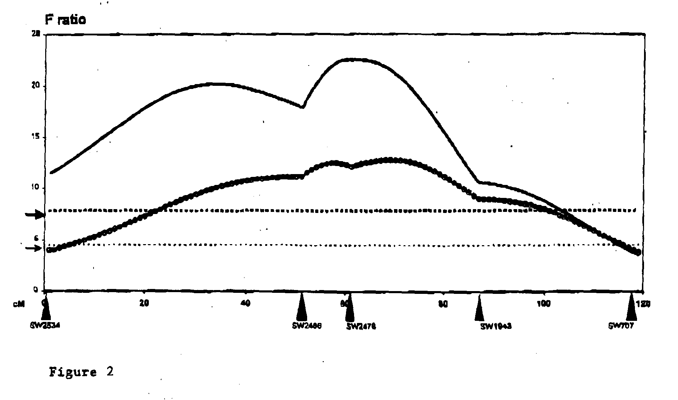

The female genetic map calculated from our data (Fig. 2) shows the same marker order but differences in distances between markers compared to the map of Rohrer et al. (1996). An increase in length of roughly 10cM (Kosambi) is observed between markers SW2534 and SW2456 (37.8cM versus 27.6cM) as well as between SW2476 and SW1943 (21.5cM versus 9.8cM). However, a smaller distance is observed on our map in the interval between SW2456 and SW2476 (9.4cM versus 19.8cM). Sensitivity analysis did not point towards specific families causing these differences (data not shown). As the mapping population of Rohrer et al. (1996) consisted of 94 F2 animals and only female parents contribute mapping information, the recombination frequencies are expected to be rather rough estimates and might explain the differences observed.

Figure 2 shows the test statistics of the QTL analysis for IMF and BFT and the threshold levels along the genetic map of the X chromosome. For both fatness traits, a genomewise significant (p<5%) QTL is detected. The estimated best position of the QTLs is in different but neighbouring marker intervals at 60cM for BFT and 69 cM for IMF (Table 2). As expected, the alleles from the obese Meishan breed increase the amount of intramuscular fat as well as back fat. The estimated size of the QTL effect is smaller in females than in males for both traits (Table 2).

In summary, this research reveals a number of Mendelian inheriting traits and at least two chromosomal areas to which imprinted genes had so far not been mapped in pigs, and the known homologies to humans and mice do not reveal obvious positional imprinted candidates. Results indicate new areas subject to imprinting that affect body composition. Although obesity genes reported in humans and mice map to homologous regions of the imprinted QTLs found in this study¹³, imprinting has only been reported for the Prader-Willi Syndrome (HSA15q11.2-q12). According to our study, imprinting might be more common in development of obesity than currently anticipated.

For the practice of animal breeding, identification of major imprinted loci affecting body composition has several implications. Our results call for a revision of the breeding evaluation methods that are based on the assumption of a large number of genes acting additively. Identification of imprinted loci opens new perspectives for crossbreeding which is common practice in pig breeding. Imprinted genes could further accommodate the differentiation between sow lines, which are required to have optimal body composition to support their reproductive performance, and boar lines, which ensure high quality pork.

Although the mechanisms underlying imprinting are not totally unravelled¹⁴, this study clearly demonstrates the important role of imprinting for body composition traits. Statistical testing for imprinting should become a standard procedure in human and animal genetic research, both in genome scans and in evaluating candidate genes. The invention for example finds its use in present pig breeding programmes with pure breeding for additive genetic progress and crossbreeding for crossbreeding (dominance) effects (heterosis) in the final breeding product, are based on mendelian inheritance. Generally, in sire lines selection is focussed on production and meat quality traits like daily gain, backfat thickness (BF) muscle depth (MD) and intramuscular fat (IMF), while in dam lines selection is focussed on fertility traits. Selection is only on fertility in the dam lines because the genetic background for fertility is mainly relevant in CD sows (90% of total sow population). Selection for daily gain, backfat and carcass traits is mainly performed in sire lines. Due to short generation interval in these lines, additive genetic progress is best reachable in these lines. Although the slaughter pig receives the genes for traits like backfat thickness and daily gain both from its dam and sire, selection for these traits is limited in dam lines because of negative genetic relationships between fertility and production traits.

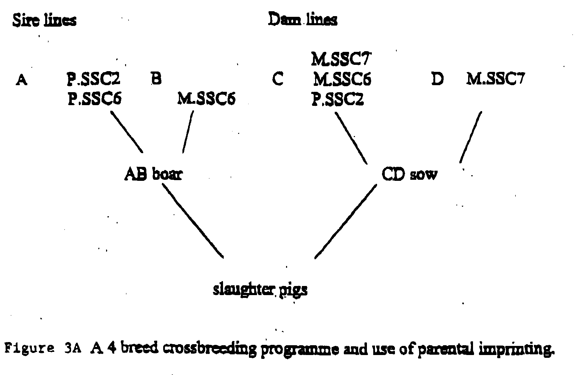

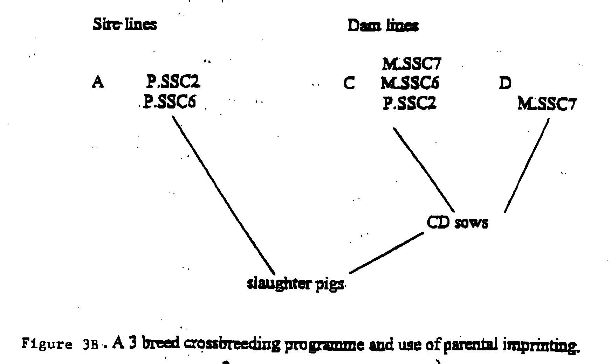

The use of parental imprinting will change the set up of pig breeding programmes and improve its efficiency enormously. New and economically relevant applications of parental imprinted markers/genes for BF and IMF are: for example shown in figure 3A
1. Paternally expressed markers/genes for BF enable the possibility to select a dam (eg C) line for thick backfat, with expression of these genes in the CD-sows but without expression of these genes in the slaughter pig. By this procedure the favourable biological relationship between fertility and backfat can be used without having 'fat' slaughterpigs.
2. Paternally expressed markers/genes for IMF enable also the set up of a sire line (eg A) with a high genetic level of IMF but with 2 different applications:
   a) the use of the sire line as a purebred boar (A in fig. 3B) will result in slaughter pigs with high IMF
   b) the use of sire line as the dam of a crossbred boar (BxA in fig. 3A) will result in slaughter pigs without the high IMF-level.

The invention is in general applicable to purebred sire lines selection on paternally expressed genes/markers which need to be expressed in the slaughter pig and to purebred dam lines selection on maternal expressed genes/markers which need to be expressed in the slaughter pig.

### References

1. Nezer, C. et *al. Nature Genet.* **21**, 155-156 (1999)
2. Jeon, J.-T. *et al. Nature Genet.* **21**, 157-158 (1999).
3. Janss, L. L. G., Van Arendonk, J. A. M. & Brascamp, E. W. *Genetics* **145,** 395-408 (1997).
4. De Koning, D. J. *et al. Genetics* **152,** 1679-1690 (1999).
5. Knott, S.A. *et al. Genetics* **149,** 1069-1080 (1998).
6. Goureau, A. *et al. Genomics* **36**, 252-262 (1996).
7. Rettenberger, G. *et al*. *Genomics* **26**, 372-378 (1995).
8. Pearsall, R.S. *et al. Mamm. Genome* **9***,* 261-262 (1998).
9. Hawken, R.J. *et al. Mamm. Genome* **10**, 824-830 (1999).
10. Chardon, P., Renard, C. *&* Vaiman, M. *Immunol. Rev.* **167,** 179-192 (1999).
11. Morison, I. M. & Reeve, A. E. *Hum. Mol. Genet.* **7,** 1599-1609 (1998).
12. Gerbens, F., Rettenberger, G., Lenstra, J. A., Veerkamp, J. H. & Te Pas, M. F. *Mamm*. *Genome* **8,** 328-332 (1997).
13. Perusse, L. *et al. Obesity Res.* **7,** 111-129 (1999).
14. Constancia, M., Pickard, B., Kelsey, G. & Reik, W. *Genome Res.* **8**, 881-900 (1998).
15. Rattink et al., 1999

### Figure legends

**Fig. 1** Test statistic profiles for three porcine chromosomes that exhibit imprinting effects for one of the body composition traits. The black line represents the test statistic for a Mendelian QTL vs. H₀ of no QTL. The blue line represents the test statistic for a paternally expressed QTL vs. no QTL. The red line represents the test statistic for a maternally expressed QTL vs. no QTL. The black horizontal line denotes the 5% genomewise threshold for the Mendelian model and the blue line indicates the same threshold for the imprinting model. **a**, SSC2 and backfat thickness; **b**, SSC6 and intramuscular fat content; **c**, SSC7 and muscle depth; **d**, SSC7 and backfat thickness. Homologous human regions are indicated as colored bars (refs. 6, 7, 8 and
   http://www.toulouse.inra.fr/lgc/pig/cyto/cyto.htm). Imprinted genes located within these human chromosomal areas are underneath. Alignment of the porcine cytogenetic and genetic map is adapted from
   http://sol.marc.usda.gov/genome/swine/htmls/chromosome_list.h tml.
**Fig. 2.** Test statistic profile for chromosome X.
   BFT is indicated as a solid line, IMF is shown as a circled line. 5% genomewise significant threshold determined by permutation is shown as a dotted line.
**Fig. 3A** A 4 breed crossbreeding programme and use of parental imprinting
**Fig. 3B** A 3 breed crossbreeding programme and use of parental imprinting

**Table 1**

| QTL analysis for three body compostion traits under different genetic models | | | | | |
|---|---|---|---|---|---|
| Chromosome | Genetic model^{a} | Test statistic ^{b} | Position ^{c} | Confidence interval^{d} | QTL effect (s.e.)^{e} |
| 2 | Mendelian | 10.72^{**} | Backfat thickness mm. 36 | 12-82 | 1.28(0.28 0.34(0.43 |
| 2 | Paternal | 23.51^{***} | 36 | 0-73 | 0.94(0.19) |
| 2 | Maternal | 2.47 | 44 | N/A | 0.30(0.19) |
| 7 | Mendelial | 41.05^{***} | 57 | 38-72 | -2.30(0.25)0.09 |
| 7 | Paternal | 33.11^{***} | 61 | N/A | -1.09(0.19) |
| 7 | Maternal | 51.31^{***} | 56 Muscle depth mm. | N/A | -1.35(0.19) |
| 7 | Mendelian | 25.00^{***} | 59 | 40-70 | -2.21(0.32 0.85(0.50 |
| 7 | Paternal | 7.36 | 61 | N/A | -0.66(0.24) |
| 7 | Maternal | 51.46^{***} | 56 Intramuscular fat content % | 41-72 | -1.71(0.24) |
| 6 | Mendelian | 6.90 | 122 | 101-165 | -0.17(0.05 0.01(0.07 |
| 6 | Paternal | 14.68^{*} | 117 | 90-150 | -0.13(0.03) |
| 6 | Maternal | 14.50^{*} | 23 | 0-52 | 0.14(0.04) |
| ^{*}p <0.05. ^{***}p <0.01. ^{***}p<0.001 ^{a}Mendilian refers to a model where an additive and a dominance effect are estimated⁴; Paternal and Maternal refer to models where only a paternally or a maternally expressed QTL effect are estimated. ^{b}Test statistics for the different models versus the H₀ of no QTL. The risk levels are on a genomewide level and estimated by permutations⁴.^{c}Most likely position of a QTL in Haldance cM. ^{d}Empirical confidence intervals obtained by bootstrapping. From each of 10,000 bootstrap replicates the best test statistic was stored. The 95% cut-off point of the sorted (in descending order) test statistics provides an empirical threshold for the confidence interval. Confidence intervals were calculated for the standard model and for the imprinting models when relevant. ^{e}Estimates of additive and dominance effect under the standard model and of the parentally expressed allele under the imprinting models. The additive and the parental effect are expressed as the deviation of the Meishan allele. The dominance effect is expressed as the deviation of the heterozygotes from the mean of the two groups of homozygotes⁴. Standard errors of the estimates are given in parentheses. | | | | | |

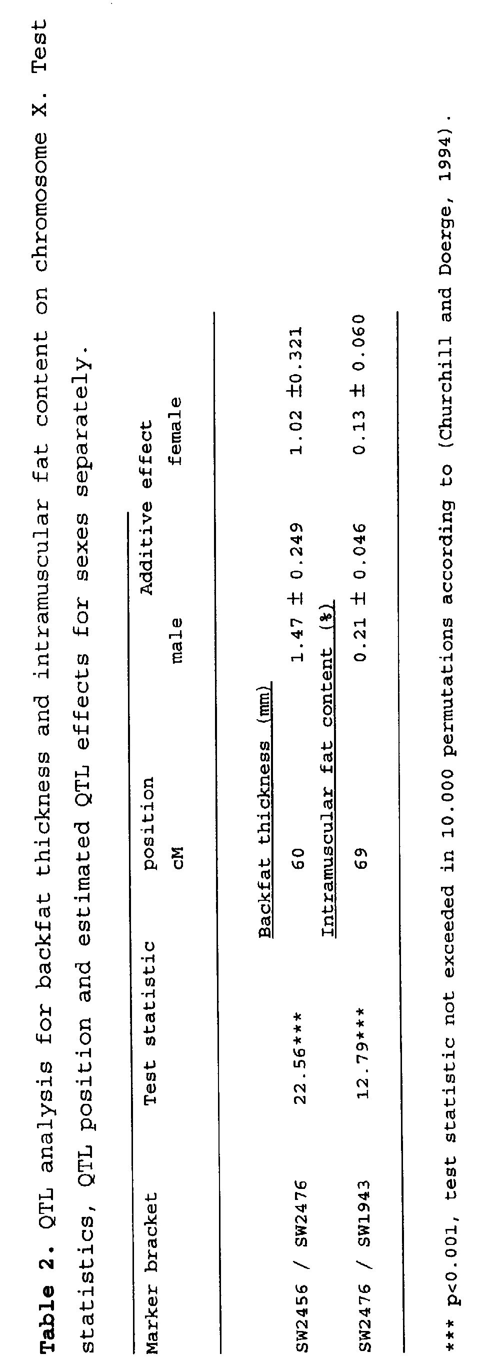

## Claims

1. An isolated and/or recombinant nucleic acid comprising a sequence affecting the quantitative trait muscle depth in mammals, said sequence being derived from a locus corresponding to region HSA6p21.3-p.22 in humans and corresponding to the homologous region on ssc7 in pigs.

2. An isolated and/or recombinant nucleic acid according to claim 1, wherein said quantitative trait is maternally expressed.

3. An isolated and/or recombinant nucleic acid comprising a sequence affecting the quantitative trait back fat thickness in mammals, said sequence being derived from a locus corresponding to a region on ssc7 in pigs, which region overlaps with the region identified in claim 1 affecting the quantitative trait muscle depth on ssc7 in pigs.

4. An isolated and/or recombinant nucleic acid comprising a sequence affecting the quantitative trait back fat thickness in mammals, said sequence being derived from a locus corresponding to a region on ssc2 of pigs which maps about 3 cM away from the IGF2 region.

5. An isolated and/or recombinant nucleic acid according to claim 4, wherein said quantitative trait is paternally expressed.

6. An isolated and/or recombinant nucleic acid comprising a sequence affecting the paternally imprinted quantitative trait intramusccular fat in mammals, said sequence being derived from a locus corresponding to a region on the long arm of ssc6 of pigs.

7. An isolated and/or recombinant nucleic acid comprising a sequence affecting the maternally expressed quantitative trait intramusccular fat in mammals, said sequence being derived from a region HSA6q22-ter in humans and corresponding to the homologous on the short arm of ssc6 of pigs.

8. An isolated and/or recombinant nucleic acid comprising a sequence affecting the quantitative trait intramusccular fat in mammals, said sequence being derived from a region localised at about 60cM on the X chromosome in pigs.

9. An isolated and/or recombinant nucleic acid comprising a sequence affecting the quantitative trait back fat thickness in mammals, said sequence being derived from a region localised at about 69cM on the X chromosome in pigs.

10. An isolated and/or recombinant nucleic acid according to any one of claims 1-9 which is a specific probe or primer for a quantitative trait.

11. An isolated and/or recombinant nucleic acid comprising a sequence capable of identifying a quantitative trait as identified in any one of claims 1-9.

12. An isolated and/or recombinant nucleic acid according to claim 10 or 11, which comprises a microsatellite marker.

13. Use of an isolated and/or recombinant nucleic acid acoording to any one of claims 1-12 in identifying a quantitative trait locus in a mammal.

14. Use according to claim 13, wherein said quantitative trait locus is subject to genomic imprinting.

15. Use of an isolated and/or recombinant nucleic acid acoording to any one of claims 1-12 for selection of a domestic animal having desired genetic properties.

16. Use according to claim 15, wherein said domestic animal is a breeding animal or an animal for slaughter.

17. Method for breeding a population of domestic animals having desired genetic properties, comprising testing animals to be cross-bred with at least one nucleic acid according to any one of claims 1-12, for the presence of a desired allele of a quantitative trait.

18. A method according to claim 17, wherein said testing comprises contacting a sample from an animal to be cross-bred with a nucleic acid according to any one of claims 1-12 and detecting the presence or absence of hybridisation.

19. A mammalian cell comprising a recombinant nucleic acid according to any one of claims 1-9.

20. A transgenic animal comprising at least one cell according to claim 19.
